# EUROPEAN PATENT APPLICATION

(11) **EP 4 420 697 A1**
(43) Date of publication of application: **28.08.2024**
(21) Application number: 23158597.7
(22) Date of filing: 24.02.2023
(51) Int. Cl.: A61M 5/315, A61M 5/20, A61M 5/32

(54) **END-OF-DOSE INDICATOR FOR DRUG-DELIVERY DEVICE**

(71) Applicant: Becton, Dickinson and Company, Franklin Lakes, NJ 07417-1880 (US)
(72) Inventor: BESSON, Nicolas, 38650 Treffort (FR); PLOUVIER, Adrien, 38400 SAINT MARTIN D'HERES (FR); DUFOUR, Angeline, 38800 Le Pont-de-Claix (FR)
(74) Representative: Germain Maureau

(57) **Abstract**

Provided herein is a drug delivery device including a housing, a drive assembly including a plunger body having a pre-use position, an injection position, and a post-use position, and a resiliently-biased audio indicator member connected to the distal end of the plunger body, wherein the housing includes at least one rib extending in a longitudinal direction of the device and projecting radially inward from the housing into the housing interior, the at least one rib having a plurality, each projecting inward a different distance, the audio indicator member engaging the at least one rib when the plunger body is in the injection position to deflect the audio indicator member from an unbiased position to a biased position, the audio indicator member disengaging from the rib and contacting a portion of the housing when the plunger body reaches the post-use position to cause an audible indication.

## Description

### BACKGROUND OF THE INVENTION

### Field of the Invention

The present disclosure relates generally to a drug delivery device and, more specifically, to an auto-injector.

### Description of Related Art

Various types of automatic injection devices have been developed to allow drug solutions and other liquid therapeutic preparations to be administered by untrained personnel or to be self-injected. Generally, these devices include a reservoir that is pre-filled with the liquid therapeutic preparation and some type of automatic needle-injection mechanism that can be triggered by the user. Many of these devices, such as auto-injectors, are designed so that the reservoir, such as a pre-filled syringe, is assembled into the device during assembly of the device. In addition to automatically deploying the needle-injection mechanism, many drug delivery devices also automatically shield the needle after use of the device to prevent any unintended contact with the needle.

To aid users of such injection devices, it can be helpful to include indicia indicating that a dose of therapeutic preparation has been delivered. However, many known solutions are inadequate, resulting in indications that are inaudible, or breakage of components and thus premature indications attributed to the noise associated with breakage or no indication at all. Accordingly, there is a need in the art for more robust audible indicators of completed drug delivery.

### SUMMARY OF THE INVENTION

Provided herein is a drug delivery device including a housing having a proximal end, a distal end, and defining an interior, a syringe having a barrel, a stopper, and a needle, at least a portion of the syringe positioned within the housing interior, a drive assembly arranged at least partially in the housing interior and including a plunger body configured to move the stopper within the barrel upon actuation of the drive assembly, the plunger body having a pre-use position prior to movement of the plunger body, an injection position where the plunger body moves the stopper within the barrel, and a post-use position where movement of the plunger body has stopped, and a resiliently-biased audio indicator member connected to the distal end of the plunger body, wherein the housing includes at least one rib extending in a longitudinal direction of the device and projecting radially inward from the housing into the housing interior, the at least one rib having a plurality of sections, each of the plurality of sections projecting inward a different distance, the audio indicator member engaging the at least one rib when the plunger body is in the injection position to deflect the audio indicator member from an unbiased position to a biased position, the audio indicator member disengaging from the rib and contacting a portion of the housing when the plunger body reaches the post-use position to cause an audible indication of a transition of the plunger body from the injection position to the post-use position.

### BRIEF DESCRIPTION OF THE DRAWINGS

**FIG. 1** is a perspective view of a drug delivery device according to non-limiting embodiments described herein;
**FIG. 2** is a cross-sectional view of a drug delivery device according to non-limiting embodiments described herein;
**FIGS. 3A-3B** are perspective views of a plunger body and audio indicator member of a drug delivery device according to non-limiting embodiments described herein;
**FIGS. 4A-4B** are cross-sectional and enlarged views of a drug delivery device and rib according to non-limiting embodiments described herein; and
**FIGS. 5A-5B** are perspective views of a drug delivery device according to non-limiting embodiments described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of" 1 to 10" is intended to include all sub-ranges between (and including) the recited minimum value of 1 and the recited maximum value of 10, that is, having a minimum value equal to or greater than 1 and a maximum value of equal to or less than 10.

International Patent Application Publication Nos. WO 2020/173991, WO 2020/173992, WO 2020/173993, WO 2020/173994, and WO 2020/173995 are incorporated herein by reference in their entirety.

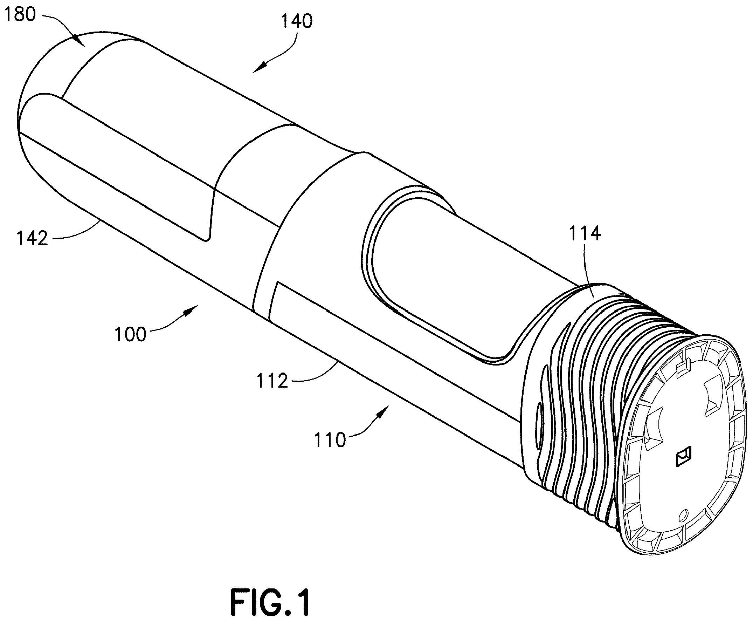

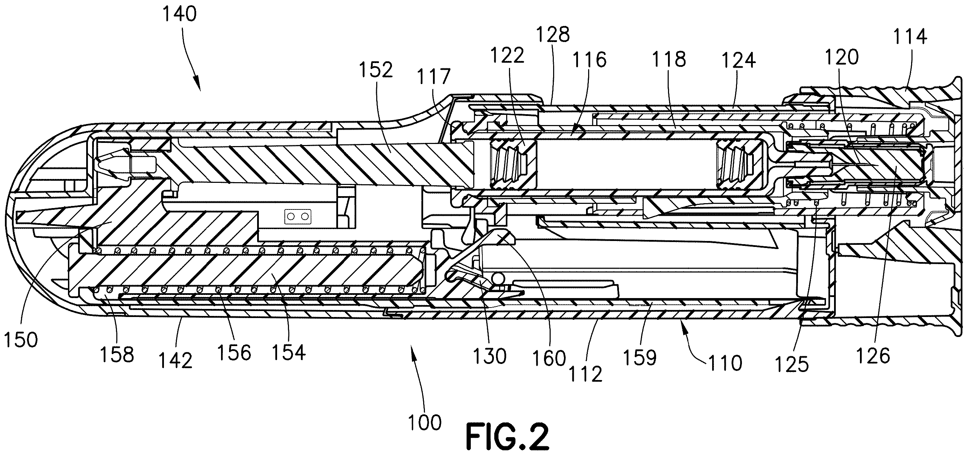

Turning to FIGS. 1 and 2, shown is a drug delivery device 100. Drug delivery device 100 may include a first subassembly 110, a second subassembly 140, and a syringe 116. The first subassembly 110 may include a lower housing shell 112, a cap 114, a syringe holder 118, a needle cover 124, and a cassette body 128. The second subassembly 140 may include a drive assembly including a plunger body 150, the plunger body 150 including a plunger rod 152 and a spring guide member 154 including a drive member 156 and a drive opening 158. Second subassembly 140 may further include a motor body 159, a lever actuation member 130, and an upper housing shell 142. The syringe 116 may be received by the syringe holder 118 and includes a barrel 117, a stopper 122, a needle 120, and a rigid needle shield (RNS) 126. Although an RNS is utilized, other suitable needle shield arrangements may be utilized. The lower housing shell 112, the cassette body 128, and the upper housing shell 142 generally form a housing for receiving the various components of the drug delivery device 100, although other suitable housing arrangements may be utilized. The first subassembly 110 and the second subassembly 140 may be secured to each other during assembly by a locking clip, although other suitable arrangements may be utilized. The drug delivery device 100 may be an auto-injector, although the features described herein may be incorporated into other suitable drug delivery devices.

Drug delivery device 100 is configured to automatically deliver a dose of medicament from the syringe 116 to a patient upon actuation of the drug delivery device 100. More specifically, upon actuation of the drug delivery device 100, the drive assembly is configured to engage the stopper 122 of the syringe 116, displace the syringe 116 such that the needle 120 pierces the skin of the patient, and displace the stopper 122 within the barrel 117 of the syringe 116 to deliver the medicament within the barrel 117. The drug delivery device 100 includes a storage position, a pre-use position, an actuation position, an injection position, and a post-use position, as described in International Patent Application Publication Nos. WO 2020/173991, WO 2020/173992, WO 2020/173993, WO 2020/173994, and WO 2020/173995.

Needle cover 124 is configured to shield the needle 120 of the syringe 116 from the patient when the drug delivery device 100 is in the pre-use and the post-use positions. In particular, the needle cover 124 is moveable between a pre-use position, an actuation position, and a post-use positon, with a spring 125 biasing the needle cover 124 towards the pre-use position and the post-use position. The spring 125 may be positioned between the needle cover 124 and the syringe holder 118, although other suitable arrangements may be utilized. The lever actuation member 130 is moveable between a locked position where movement of the drive assembly is prevented and a released position where movement of the drive assembly is allowed. More specifically, the lever actuation member 130 is rotatable about a rotation axis between the locked position and the released position. When the lever actuation member 130 is in the locked position, the lever actuation member 130 is engaged with the motor body 159 and the drive assembly to prevent movement of the drive assembly.

When the lever actuation member 130 is in the released position, which may be achieved by pressing the needle cover 124 onto the patient's skin at the site of injection, shifting the needle cover proximally into the lower housing shell 112, the lever actuation member 130 is disengaged from the motor body 159 thereby allowing movement of the drive assembly toward the syringe 116. The rotation axis of the lever actuation member 130 extends perpendicular to a longitudinal axis of the drug delivery device 100, although other suitable arrangements may be utilized.

Referring again to FIGS. 1 and 2, the drive assembly includes a plunger body 150 having a plunger rod portion 152 and a drive member 156. The drive member 156 may be a compression spring received within a drive opening 158 defined by the plunger body 150, although other suitable drive members may be utilized, including, but not limited to, compressed gas, an electric motor, hydraulic pressure, other types of springs, etc. The drive member 156 engages the plunger body 150 and the motor body 159 and biases the plunger body 150 in a direction extending from the second subassembly 140 toward the first subassembly 110. The plunger body 150 defines a lever opening that receives the lever actuation member 130 and defines the rotation axis of the lever actuation member 130. The lever actuation member 130 prevents movement of the plunger body 150 when the lever actuation member 130 is in the locked position through engagement of the lever actuation member 130 with the motor body 159. Upon rotation of the lever actuation member 130 from the locked position to the released position, the lever actuation member 130 is disengaged from the motor body 159 thereby allowing the drive member 156 to move the plunger body 150 and the plunger rod 152 toward the first subassembly 110. The plunger rod 152 and the drive member 156 are spaced from and parallel to each other and extend in a longitudinal direction of the drug delivery device 100.

The drive assembly further includes a spring guide member 154 secured to the upper housing shell 142 and received within the drive opening 158 of the plunger body 150. The drive member 156 is received by the spring guide member 154 such that the drive member 156 is positioned between the plunger body 150 and the spring guide member 154. The drive assembly may also include a plunger rod cover that receives the plunger rod 152 of the plunger body 150. The plunger rod cover may be configured to guide insertion of the plunger rod 152 into the barrel 117 of the syringe 116 and engage the stopper 122 to dispense the medicament from the barrel 117 of the syringe 116. The plunger rod cover and the plunger rod 152 may be formed integrally or formed as separate components.

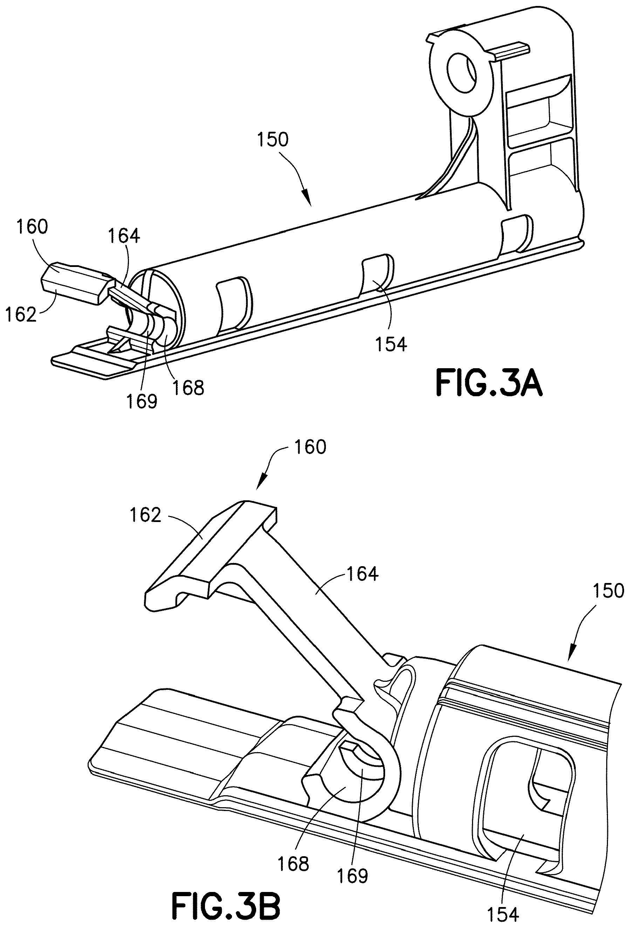

Turning to FIGS. 3A-3B, the plunger body 150 may also define a lever opening 168 at a distal end thereof that receives the lever actuation member 130 and defines the rotation axis of the lever actuation member 130. The plunger body 150 may also include a lever rib 169 extending into the lever opening 168 of the plunger body 150. The lever rib 169 is configured to be received by the lever actuation member 130. In non-limiting embodiments, engagement between the lever actuation member 130 and the lever rib 169 prevents relative lateral movement between the plunger body 150 and the lever actuation member 130.

With continuing reference to FIGS. 3A-3B, the plunger body 150 of the drive assembly may also include an audio indicator member 160, optionally at distal end thereof, configured to provide an audible indication to a user when the drug delivery device 100 transitions to the post-use position. Audio indicator member 160 may be configured and arranged such that the lever opening 168 and lever rib 169 described above are adjacent to, or formed as part of, audio indicator member 160. As will be described below, the audio indicator member 160 may be configured to engage one or more ribs of the lower housing shell 112, optionally arranged on and/or as part of cassette body 128, when the drug delivery device 100 is in the injection position, thereby deflecting the audio indicator member 160. When the drug delivery device 100 transitions from the injection position to the post-use position, the audio indicator member 160 may disengage from the rib(s) and contact the lower housing shell 112 and/or the cassette body 128 to provide an audible click, although the audio indicator member 160 could also contact other suitable portions of the drug delivery device 100 to provide the audible indicator. In non-limiting embodiments, audio indicator member 160 is arranged in a t-shaped member, having a head portion 162 arranged perpendicularly to an arm portion 164. Arm portion 164 may be arranged to extend from plunger body 150 at an angle relative to the longitudinal axis of plunger body 150, optionally to reduce and/or eliminate the likelihood of the arm portion 154 breaking or otherwise being damaged during deflection as the device is actuated. In non-limiting embodiments, arm portion 164 may be resiliently biased, such that, as described above, when the drug delivery device 100 is in the injection position, the audio indicator member 160 is deflected by the one or more ribs. When the drug delivery device 100 transitions from the injection position to the post-use position, the audio indicator member 160 may disengage from the rib(s) and return to its original orientation.

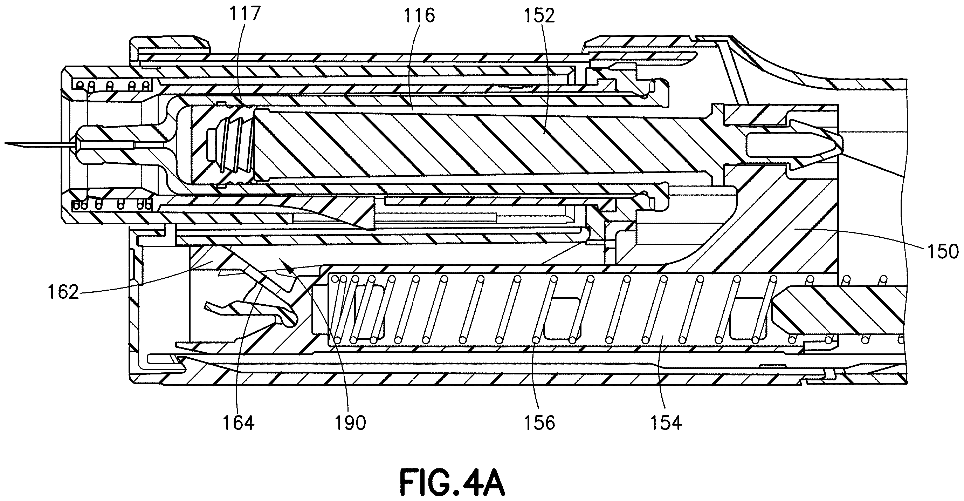

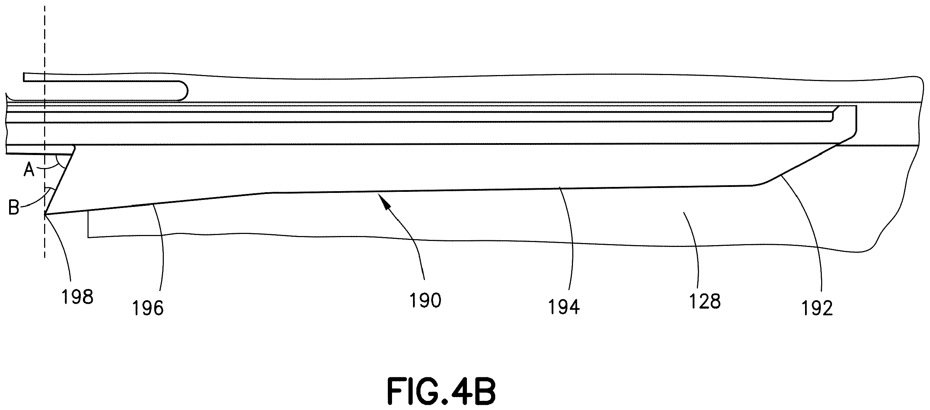

Turning to FIGS. 4A-4B, shown is a non-limiting embodiment of rib(s) 190 with which audio indicator member 160 may interact to deflect audio indicator member 160 to provide the audible indicator. As shown in greater detail in FIG. 4B, rib(s) 190 may, in non-limiting embodiments include a plurality of portions. While the illustrated non-limiting embodiment of FIGS. 4A-4B includes three distinct portions 192, 194, 196, those of skill will appreciate that rib(s) 190 may be configured to include more or fewer portions. Each portion 192, 194, 196 has a slope and, in non-limiting embodiments, the slope of each portion is distinct from the slope of each other portion. In non-limiting embodiments, an overall slope of rib(s) 190 is smooth, and/or a slope of each portion 192, 194, 196, is smooth, so as to not impair the injection in any manner. In non-limiting embodiments, a slope of first portion 192 is about 60 degrees. In non-limiting embodiments, first portion 192 has a slope that is larger than a slope of second portion 194, e.g., to maximize deflection of audio indicator member 160. In non-limiting embodiments, the slope of first portion 192 is configured to be the largest of all three portions, because during the initial stages of device activation, the force of drive member 156 is the greatest and can easily deflect audio indicator member 160 along a larger slope. In non-limiting embodiments, the slope of second portion 194 is configured to provide a deflecting force to audio indicator member 160 during device actuation and distal displacement of plunger body 150, while limiting and/or eliminating risk of breakage of the audio indicator member 160. In non-limiting embodiments, the slope of third potion 196 has a slope that is larger than the slope of second portion 194. In non-limiting embodiments, the slope of first portion 192 and the slope of third portion 196 are equal and, in other non-limiting embodiments, the slope of first portion 192 and the slope of third portion 196 are not equal. In non-limiting embodiments, a slope of one or more of portions 192, 194, 196 is constant along its respective length and, in other non-limiting embodiments, a slope of one or more of portions 192, 194, 196 is not constant along its respective length. In non-limiting embodiments, a slop of third portion 196 is the smallest of the three portions 192, 194, 196, such that the end of the injection is not impaired in any manner.

With continuing reference to FIG. 4B, rib 190 includes a distal end 198, which defines a transition from rib 190 to lower housing shell 112 and/or cassette body 128. In non-limiting embodiments, distal end 198 is arranged such that, for example as shown in FIG. 4B, an angle A, or an angle B, between rib 190 and lower housing shell 112 and/or cassette body 128, is less than 90°, thereby reducing and/or eliminating risk of the impact of audio indicator member 160 with lower housing shell 112 and/or cassette body 128 being lessened by interference between rib 190 and audio indicator member 160. In non-limiting embodiments, both angles A and B are less than 90°. In non-limiting embodiments, angle A is at least 80 degrees and angle B is at least 20 degrees, such that contact with the bottom of rib 190 may be avoided.

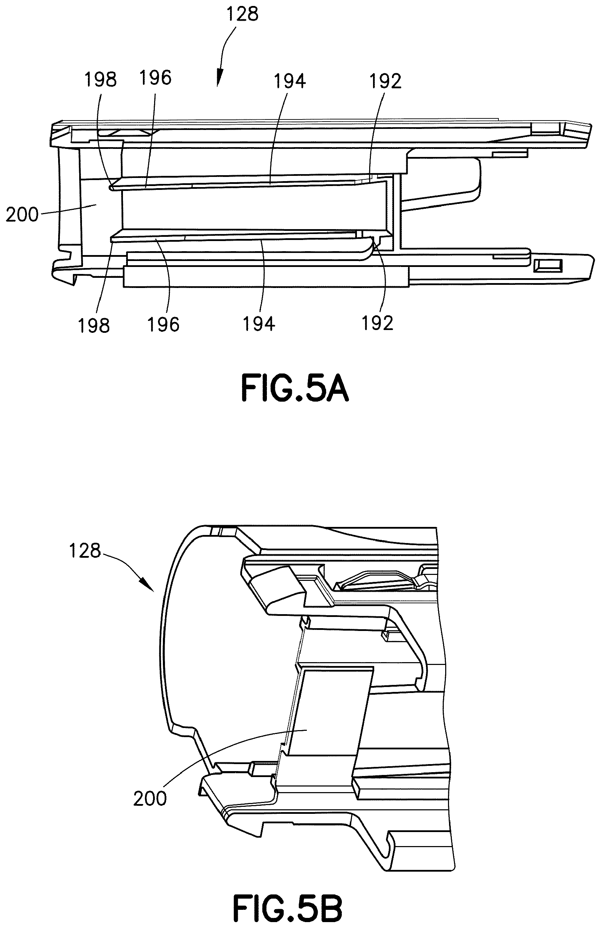

With reference to FIG. 5A, shown is a non-limiting embodiment of rib(s) 190, where a plurality of ribs are provided on cassette body 128. Those of skill will appreciate that more than two ribs 190 may be utilized, though in the non-limiting embodiment of FIG. 5A, two ribs 190 are included. In the non-limiting embodiment of FIG. 5A, ribs 190 are arranged spaced from one another, such that head portion 162 of audio indicator member 160 engages both ribs as plunger body 150 is displaced, distally during device activation. Turning to FIG. 5B, in non-limiting embodiments, drug delivery device 100 may include a strike plate 200, which is contacted by audio indicator member 160 as the drug delivery device 100 transitions from the injection position to the post-use position. Strike plate 200 may be formed on lower housing shell 112 and/or cassette body 128. Strike plate 200 may be formed of any useful material, including metal(s). In non-limiting embodiments, strike plate 200 is fixed to the cassette body 128 and/or lower housing shell 112, thereby maximizing vibration and sound level of the audio indicator member 160. In non-limiting embodiments, audio indicator member 160 may be configured such that following contact between head portion 162 and strike plate 200, head portion 162 is spaced from strike plate, thereby reducing or eliminating any dulling effect of contact between the components. In non-limiting embodiments, audio indicator member 160 is configured to provide this functionality based on a length of 164 and/or a resiliency of arm portion 164.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A drug delivery device comprising:
a housing having a proximal end, a distal end, and defining an interior;
a syringe comprising a barrel, a stopper, and a needle, at least a portion of the syringe positioned within the housing interior;
a drive assembly arranged at least partially in the housing interior and comprising a plunger body configured to move the stopper within the barrel upon actuation of the drive assembly, the plunger body having a pre-use position prior to movement of the plunger body, an injection position where the plunger body moves the stopper within the barrel, and a post-use position where movement of the plunger body has stopped; and
a resiliently-biased audio indicator member connected to the distal end of the plunger body,
wherein the housing comprises at least one rib extending in a longitudinal direction of the device and projecting radially inward from the housing into the housing interior, the at least one rib comprising a plurality of sections, each of the plurality of sections projecting inward a different distance, the audio indicator member engaging the at least one rib when the plunger body is in the injection position to deflect the audio indicator member from an unbiased position to a biased position, the audio indicator member disengaging from the rib and contacting a portion of the housing when the plunger body reaches the post-use position to cause an audible indication of a transition of the plunger body from the injection position to the post-use position.

2. The drug delivery device of claim 1, wherein the audio indicator member has a released position, the audio indicator member positioned in the biased position when the plunger body is in the injection position, the audio indicator member positioned in the released position when the plunger body is in the post-use position.

3. The drug delivery device of claim 1 or claim 2, wherein the audio indicator member is positioned in the unbiased position when the plunger body is in the pre-use position.

4. The drug delivery device of any of claims 1-3, wherein the housing comprises a lower housing shell, an upper housing shell, and a cassette body received by the lower housing shell, wherein the ribs are arranged on the cassette body.

5. The drug delivery device of claim 4, wherein the cassette body comprises an indicator platform, the audio indicator member engaging the indicator platform to provide the audible indication.

6. The drug delivery device of any of claims 1-5, wherein the at least one rib comprises two ribs separated from each other by a static distance along the longitudinal direction of the device.

7. The drug delivery device of claim 6, wherein the two ribs each comprise a first portion having a first slope, a second portion having a second slope, a third portion having a third slope, and a distal end.

8. The drug delivery device of claim 7, wherein the first slope, second slope, and third slope are all different.

9. The drug delivery device of claim 7 or claim 8, wherein the second slope is less than the first slope.

10. The drug delivery device of any of claims 7-9, wherein third slope is greater than the second slope.

11. The drug delivery device of any of claims 7-10, wherein the third slope is less than the first slope.

12. The drug delivery device of any of claims 7-11, wherein the rib, at the distal end thereof, forms an angle with the distal end of the housing that is less than 90°.

13. The drug delivery device of any of claims 1-12, wherein the audio indictor member is formed integrally with the plunger body.

14. The drug delivery device of any of claims 1-13, wherein the audio indicator member comprises a t-shape.

15. The drug delivery device of any of claims 1-14, further comprising:
a lever actuation member received within the housing interior and moveable between a locked position where movement of the drive assembly is prevented and a released position where movement of the drive assembly is allowed,
wherein the audio indicator member comprises an opening configured to receive the lever actuation member and allow rotation thereof between the locked position and the released position.
